# EUROPÄISCHE PATENTSCHRIFT

(11) **EP 2 004 838 B1**
(45) Veröffentlichungstag und Bekanntmachung des Hinweises auf die Patenterteilung: **13.06.2012**
(21) Anmeldenummer: 07724138.8
(22) Anmeldetag: 11.04.2007
(51) Int. Cl.: C12P 33/02, C12P 33/16, C12P 41/00

(54) **VERFAHREN ZUR HERSTELLUNG VON STEROIDDERIVATEN DURCH REDUKTION VON OXOSTEROIDVERBINDUNGEN ODER DURCH OXIDATION VON HYDROXYSTEROIDVERBINDUNGEN UNTER VERWENDUNG EINER HYDROXYSTEROIDDEHYDROGENASE**
PROCESS FOR THE PREPARATION OF STEROID DERIVATIVES BY REDUCTION OF OXOSTEROID COMPOUNDS OR BY OXIDATION OF HYDROXYSTEROID COMPOUNDS USING A HYDROXYSTEROID DEHYDROGENASE
PROCÉDÉ DE PRODUCTION DE DÉRIVÉS DE STÉROÏDES PAR RÉDUCTION DE COMPOSÉS OXOSTÉROÏDES OU PAR OXYDATION DE COMPOSÉS HYDROXYSTÉROÏDES EN UTILISANT UNE HYDROXYSTÉROÏDE DÉSHYDROGÉNASE

(30) Priorität: 11.04.2006 AT 6272006
(43) Veröffentlichungstag der Anmeldung: 24.12.2008
(73) Patentinhaber: IEP GmbH, 65203 Wiesbaden (DE)
(72) Erfinder: GUPTA, Antje, 65207 Wiesbaden (DE); TSCHENTSCHER, Anke, 65347 Eltville-Hattenheim (DE); BOBKOVA, Maria, 65510 Idstein (DE)
(74) Vertreter: Schwarz, Albin
(86) Internationale Anmeldenummer: PCT/EP2007/003197
(87) Internationale Veröffentlichungsnummer: WO 2007/118644

(56) Entgegenhaltungen:
- EP-A- 1 731 618
- WO-A2-2005/049816
- RIVA, S. ET AL.: "Enzymatic alpha/beta Inversion of C-3 Hydroxyl of BileAcids and Study of the Effects of Organic Solvents on Reaction Rates" JOURNAL OF ORGANIC CHEMISTRY, Bd. 53, Nr. 1, 1988, Seiten 88-92, XP002444249 in der Anmeldung erwähnt
- CARREA, G. ET AL.: "Enzymatic oxidoreduction of steroids in two-phase systems: effects of organic solvents on enzyme kinetics and evaluation of the performance of different reactors" ENZYME AND MICROBIAL TECHNOLOGY, Bd. 10, Nr. 6, 1988, Seiten 333-340, XP002444250 in der Anmeldung erwähnt
- CREMONESI, P. ET AL.: "Enzymatic Dehydrogenation of Steroids by beta-Hydroxysteroid Dehydrogenase in a Two-Phase System" ARCHIVES OF BIOCHEMISTRY AND BIOPHYSICS, Bd. 159, 1973, Seiten 7-10, XP008081450 in der Anmeldung erwähnt

## Beschreibung

Die vorliegende Erfindung betrifft ein Verfahren zur enantioselektiven enzymatischen Reduktion von Oxosteroidverdungen, wobei die Oxosteroidverbindung mit einer Hydroxysteroiddehydrogenase in Gegenwart eines Cofactors NADH oder NADPH reduziert wird.

Die vorliegende Erfindung betrifft ferner ein Verfahren zur enzymatischen Oxydation von hydroxysteroidverbindungen, wobei die Hydroxysteroidverbindung mit einer Hydroxysteroiddehydrogenase in Gegenwart eines Cofactors NAD oder NADP oxidiert wird.

Steroide sind Verbindungen die das Ringsystem des Cholesterins besitzen und sich nach Zahl der Doppelbindungen, Art, Zahl und Position funktioneller Gruppen, Anzahl der Methylgruppen, der Alkylseitenkette und der Konfiguration von Bindungen unterscheiden. Steroidverbindungen kommen sowohl im tierischen Organismus als auch in Pilzen und in Pflanzen vor und weisen vielfältige biologische Wirksamkeit auf, beispielsweise als männliche und weibliche Sexualhormone, als Hormone der Nebennieren, als Vitamine, als Gallensäuren, als Steroidsapögenine, als herzaktive Substanzen und als Krötengifte.

Unter Oxosteroidverbindungen werden nachfolgend Steroide der eingangs definierten Art verstanden, d.h. solche, die mindestens eine Ketofunktion aufweisen, wobei diese am Ringsystem oder auch an einer am Steroidgerüst befindlichen Seitenkette enthalten sein kann.

Unter Hydroxysteroidverbindungen werden nachfolgend Steroide der eingangs definierten Art verstanden, d.h. solche, die mindestens eine Hydroxyfunktion aufweisen, wobei diese am Ringsystem oder auch an einer am Steroidgerüst befindlichen Seitenkette enthalten sein kann.

Die vorliegende Erfindung betrifft ein Verfahren zur enantioselektiven enzymatischen Reduktion von Oxosteroidverbindungen der allgemeinen Formel I 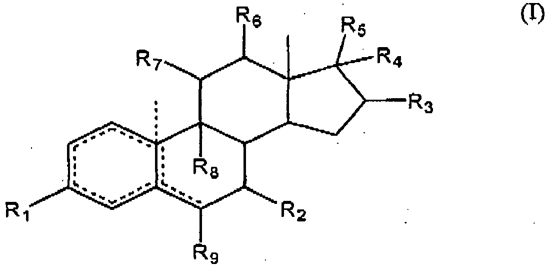 worin
R₁ Wasserstoff, eine Methylgruppe, eine Hydroxygruppe oder eine Oxogruppe,
R₂ Wasserstoff, eine Methylgruppe, eine Hydroxygruppe oder eine Oxogruppe,
R₃ Wasserstoff, eine Hydroxygruppe, eine Oxogruppe oder eine Methylgruppe,
R₄ Wasserstoff oder eine Hydroxygruppe,
R₅ Wasserstoff, ein Rest -COR₁₀, wobei R₁₀ eine unsubstituierte oder mit einer Hydroxygruppe substituierte C1-C4-Alkylgruppe ist, oder eine substituierte oder unsubstituierte C1-C4-Carboxyalkylgruppe,
oder R₄ und R₅ zusammen eine Oxogruppe,
R₆ Wasserstoff, eine Methylgruppe, eine Hydroxygruppe oder eine Oxogruppe,
R₇ Wasserstoff, eine Methylgruppe, eine Hydroxygruppe oder eine Oxogruppe,
R₈ Wasserstoff, eine Methylgruppe oder ein Halogenid, und
R₉ Wasserstoff, eine Methylgruppe, eine Hydroxygruppe, eine Oxogruppe oder ein Halogenid repräsentieren,
wobei mindestens einer von R₁, R_{2,} R₄+R₅, R₆, R₈ oder R₉ eine Oxogruppe bzw. R₅ ein Rest -COR₁₀ ist und das Strukturelement 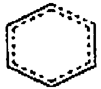
einen Benzolring oder einen C6-Ring mit 0, 1 oder 2 C-C-Doppelbindungen repräsentiert, oder der Formel II (Ketolithocholsäure)

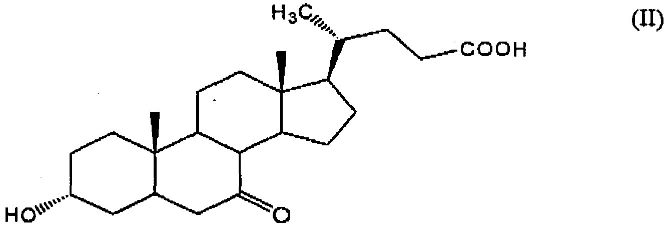

Die vorliegende Erfindung betrifft ferner ein Verfahren zur enzymatischen Oxidation von Hydroxysteroidverbindungen der allgemeinen Formel I oder von solchen, die Derivate der Gallensäure sind, wie Cholsäure, Chenodeoxchlosäure, 12-Oxocholsäure, 3-Hydroxy-12-oxocholsäure, Ketolithocholsäure und Lithocholsäure, wobei die Hydroxysteroidverbindung mit einer Hydroxysteroiddehydrogenase in Gegenwart eines Cofactors NAD oder NADP oxidiert wird.

Aufgrund der vielfältigen physiologischen Wirkungen von Steroiden ist es naheliegend, dass Steroidverbindungen und -derivate auch in großer Zahl als therapeutisch wirksame Substanzen und Arzneimittel in der Medizin zu Einsatz kommen.

So finden beispielsweise Gestagen- und Östrogenderivate weltweit verbreitete Anwendung als Kontrazeptiva; Androgene (Testosteron) werden als Anabolika und Antiandrogen beispielsweise in der Therapie von Prostatakarzinomen eingesetzt. Glucocorticoide (Cortison, Cortisol, Prednisolon und Prednison) und deren Derivate werden aufgrund ihrer antiphlogistischen, antiallergischen und immunsuppressiven Wirkung verbreitet bei der therapeutischen Behandlung von Hautkrankheiten, rheumatischen Erkrankungen, allergischen Reaktionen, Nierenerkrankungen, gastrointestinalen Erkrankungen und vielen weiteren Störungen eingesetzt.

Der Weltmarkt an biologisch aktiven Steroidverbindungen ist immens. Bei der Produktion verschiedener Steroidderivate mit unterschiedlicher Wirkung spielen Biotransformationen eine große Rolle. Dabei sind insbesondere Reaktionen die von Hydroxylasen und von Dehydrogenasen katalysiert werden von Bedeutung. Eine besondere Rolle spielen hierbei die delta-1-Dehydrogenierung, die 11 beta-Reduktion, die 20 beta-Reduktion, die 17 beta-Reduktion , stereoselektive Reduktionen an der Position 3 und 7, aber auch Oxidationen der Hydroxygruppen, insbesondere an den Positionen 3, 7, 12 und 17.

Industriell werden Bioreduktionen an Steroiden bisher ausschließlich mit ganzen, intakten Zellen und in Substratkonzentrationen von weit unter 10 g/l realisiert. Das liegt zum einen daran, dass die für die Biotransformationen verantwortlichen Enzyme bisher nicht charakterisiert bzw. exprimierbar waren, zum anderen auch daran, dass keine zufriedenstellende technologische Lösung vorlag, die einerseits das Problem der geringen Löslichkeit der Steroide in wässrigem Medium und andererseits das Problem der Regenerierung der Cofaktoren NADH und NADPH in ausreichendem Maß löst.

Oxidationen an Steroiden werden bisher industriell chemisch durchgeführt.

Versuche der enantioselektiven Reduktion von Steroiden mit isolierten Enzymen wurden 1975 bis 1988 im wesentlichen von G. Carrea beschrieben (Eur. J. Biochemistry 44, 1974 p. 401-405; Biotechnology and Bioengineering, Vol 17, 1975, p. 1101-1108; Enzyme Microb. Technol. Vol 6, July, 1984, p. 307-311; Biotechnology and Bioengineering, Vol 26, 1984, p. 560-563; J. Org. Chem. 51, 1986, p. 2902-2906; J. Org. Chem. 58, 1993, p. 499-501; J. Org. Chem., 53, 1988, p. 88-92; Enzyme Microb. Technol., Vol 10, June, p. 333-339; Archives of Biochemistry and Biophysics, 159, 1973, p. 7-10).

Hierbei wurden verschiedene Hydroxysteroiddehydrogenasen (HSHD) verwendet, wobei die Regenerierung des Cofactors NADH im wesentlichen durch Kopplung mit den Enzymen Lactatdehydrogenase, Formiatdehydrogenase oder auch Alkoholdehydrogenase aus Hefe erreicht wurde. Die Regeneration von NADP wurde mit Hilfe von Glucosedehydrogenase durchgeführt. Zur Überwindung der Löslichkeitsprobleme wurden auch Versuche im Zweiphasensystem mit Ethylacetat und Butylacetat als organische Phase durchgeführt. Auch mit isolierten Enzymen im Zweiphasensystem wurde dabei in Konzentrationsbereichen von meist weit unter 10 g/l gearbeitet, wobei die erreichten "total turn over numbers" (TTN = mol reduzierte Oxosteroidverbindung / mol eingesetzter Cofactor) ebenfalls weit unter 1000 lagen, wodurch diese Prozesse keinen wesentlichen ökonomischen Vorteil im Vergleich zu Ganzzellverfahren darstellten.

Des weiteren existieren Arbeiten, bei denen durch Kopplung von Oxidation und Reduktion die Konversion von Hydroxygruppen von 7 alpha nach 7 beta durchgeführt wurde. Dies wurde durch Kopplung von 7α HSDH und 7β HSDH erreicht (Pedrini et al., Steroids 71 (2006) p 189-198). Auch bei diesem Verfahren wurde in Konzentrationsbereichen von weit unter 10 g/l gearbeitet und die erreichten "total turn over numbers" (TTN = mol reduzierte Oxosteroidverbindung / mol eingesetzter Cofactor) lagen unter 100, wodurch auch diese Prozesse ökonomisch nicht relevant sind.

Die Erfindung bezweckt die Vermeidung dieser Nachteile und Schwierigkeiten und stellt sich die Aufgabe, ein Verfahren bereit zu stellen, dass die enantioselektive Reduktion bzw. Oxidation von Oxosteroidverbindungen bzw. Hydroxysteroidverbindungen mit höheren Umsätzen, in höheren Konzentrationsbereichen und mit höheren TTN der Cofactoren und damit in ökonomischerer Weise ermöglicht.

Diese Aufgabe wird bei einem Verfahren der eingangs genannten Art dadurch gelöst, dass
a) die Oxosteroidverbindung in einer Konzentration von ≥50 g/l im Reaktionsansatz vorliegt,
b) der durch die Hydroxysteroiddehydrogenase gebildete oxidierte Cofactor NAD oder NADP kontinuierlich durch Oxidation eines sekundären Alkohols der allgemeinen Formel R_{X}R_{Y}CHOH, wobei R_{X}, R_{Y} unabhängig voneinander verzweigtes oder unverzweigtes C1-C8-Alkyl darstellen und C_{insgesamt} ≥ 3, **oder durch Oxidation eines C4-C6-Cycloalkanols** regeneriert wird, und
c) zur Oxidation des sekundären Alkohols der allgemeinen Formel R_{X}R_{Y}CHOH bzw. des Cycloalkanols eine zusätzliche Oxidoreduktase/Alkoholdehydrogenase eingesetzt wird, wobei als Oxosteroidverbindung eine Verbindung der allgemeinen Formel I 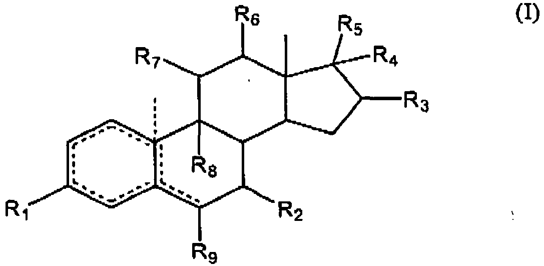 worin
   R₁ Wasserstoff, eine Methylgruppe, eine Hydroxygruppe oder eine Oxogruppe,
   R₂ Wasserstoff, eine Methylgruppe, eine Hydroxygruppe oder eine Oxogruppe,
   R₃ Wasserstoff, eine Hydroxygruppe, eine Oxogruppe oder eine Methylgruppe,
   R₄ Wasserstoff oder eine Hydroxygruppe,
   R₅ Wasserstoff, ein Rest -COR₁₀, wobei R₁₀ eine unsubstituierte oder mit einer Hydroxygruppe substituierte C1-C4-Alkylgruppe ist, oder eine substituierte oder unsubstituierte C1-C4-Carboxyalkylgruppe,
   oder R₄ und R₅ zusammen eine Oxogruppe,
   R₆ Wasserstoff, eine Methylgruppe, eine Hydroxygruppe oder eine Oxogruppe,
   R₇ Wasserstoff, eine Methylgruppe, eine Hydroxygruppe oder eine Oxogruppe,
   R₈ Wasserstoff, eine Methylgruppe oder ein Halogenid, und
   R₉ Wasserstoff, eine Methylgruppe, eine Hydroxygruppe, eine Oxogruppe oder ein Halogenid repräsentieren,
   wobei mindestens einer von R₁, R₂, R₄+R₅, R₆ R₈ oder R₉ eine Oxogruppe bzw. R₅ ein Rest -COR₁₀ ist und das Strukturelement 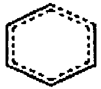
   einen Benzolring oder einen C6-Ring mit 0, 1 oder 2 C-C-Doppelbindungen repräsentiert, oder der Formel II (Ketolithocholsäure) 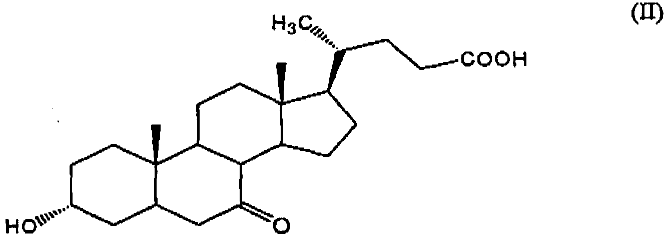 eingesetzt wird.

Die oben genannte Aufgabe wird bei einem Verfahren der eingangs genannten Art ferner dadurch gelöst, dass
a) die Hydroxysteroidverbindung in einer Konzentration von ≥50 g/l im Reaktionsansatz vorliegt,
b) der durch die Hydroxysteroiddehydrogenase gebildete reduzierte Cofactor NADH oder NADPH kontinuierlich durch Reduktion einer Ketoverbindung der allgemeinen Formel R_{X}R_{Y}CO, wobei R_{X}, R_{Y} unabhängig voneinander verzweigtes oder unverzweigtes C1-C8-Alkyl darstellen und C_{insgesamt} ≥ 3, oder durch Reduktion eines C4-C6-Cycloalkanons regeneriert wird, und
c) zur Reduktion der Ketoverbindung der allgemeinen Formel R_{X}R_{Y}CO bzw. des Cycloalkanons eine zusätzliche Oxidoreduktase/Alkoholdehydrogenase eingesetzt wird,
wobei als Hydroxysteroidverbindung die Gallensäurederivate Cholsäure, Chenodeoxycholsäure, 12-Oxocholsäure, 3-Hydroxy-12-oxocholsäure, Ketolithocholsäure und Litocholsäure oder eine Verbindung der allgemeinen Formel I eingesetzt werden, worin
R₁ Wasserstoff, eine Methylgruppe, eine Hydroxygruppe oder eine Oxogruppe,
R₂ Wasserstoff eine Methylgruppe, eine Oxogruppe oder eine Hydroxygruppe,
R₃ Wasserstoff, eine Hydroxygruppe, eine Oxogruppe oder eine Methylgruppe,
R₄ Wasserstoff oder eine Hydroxygruppe,
R₅ Wasserstoff, ein Rest -COR₁₀, wobei R₁₀ eine unsubstituierte oder mit einer Hydroxygruppe substituierte C1-C4-Alkylgruppe ist, oder eine substituierte oder unsubstituierte C1-C4-Carboxyalkylgruppe,
oder R₄ und R₅ zusammen eine Oxogruppe,
R₆ Wasserstoff, eine Methylgruppe, eine Oxogruppe oder eine Hydroxygruppe,
R₇ Wasserstoff, eine Methylgruppe, eine Oxogruppe oder eine Hydroxygruppe,
R₈ Wasserstoff, eine Methylgruppe oder ein Halogenid, und
R₉ Wasserstoff, eine Methylgruppe, eine Hydroxygruppe, eine Oxogruppe oder ein Halogenid repräsentieren,
wobei mindestens einer von R₁, R₂, R₄, R₆, R₇, R₈ oder R₉ eine Hydroxygruppe ist und das Strukturelement 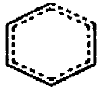 einen Benzolring oder einen C6-Ring mit 0, 1 oder 2 C-C-Doppelbindungen repräsentiert.

Die vorliegende Erfindung stellt eine wesentliche Verbesserung der enantioselektiven enzymatischen Reduktions- und Oxidationsreaktion am Steroidgerüst gegenüber dem Stand der Technik dar. Die vorliegende Erfindung ermöglicht die Reduktion bzw. Oxidation von Oxosteroidverbindungen zu den korrespondierenden Hydroxy- bzw. Oxosteroiden mit freien Enzymen in Konzentrationsbereichen, die weit über die im Stand der Technik Beschriebenen hinausgehen.

Im erfindungsgemäßen Verfahren wird als Co-Faktor NADH oder NADPH eingesetzt. Unter dem Begriff "NADP" wird Nicotinamid-adenin-dinucleotid-phosphat, unter "NADPH" reduziertes Nicotinamid-adenin-dinucleotid-phosphat verstanden. Der Begriff "NAD" bedeutet Nicotinamid-adenin-dinucleotid, der Begrif "NADH" reduziertes Nicotinamid-adenin-dinucleotid.

Vorzugsweise wird als sekundärer Alkohol der allgemeinen Formel R_{X}R_{Y}CHOH 2-Propanol, 2-Butanol, 2-Pentanol, 4-Methyl-2-pentanol, 2-Hexanol, 2-Heptanol, 5-Methyl-2-hexanol oder 2-Octanol und als Cycloalkohol Cyclohexanol eingesetzt.

Vorzugsweise wird als Keton der allgemeinen Formel R_{X}R_{Y}CO Aceton, 2-Butanon, 2-Pentanon, 4-Methyl-2-pentanon, 2-hexanol, 2-Heptanon, 5-Methyl-2-hexanon oder 2-Octanon und als Cycloalkanon Cyclohexanon eingesetzt.

Das Keton der allgemeinen Formel R_{X}R_{Y}CO bzw. das C4-C6-Cycloalkanon und der sekundäre Alkohol der allgemeinen Formel R_{X}R_{Y}CHOH bzw. der C4-C6-Cycloalkanol werden im folgenden unter dem allgemeinen Begriff Cosubstrat zusammengefasst.

Bevorzugt werden die erfindungsgemäßen Verfahren in einem wässrigen organischen Zweiphasensystem durchgeführt. Das zur Coenzymregenerierung eingesetzte Cosubstrat ist dabei zweckmäßig nicht mit Wasser mischbar und bildet somit die organische Phase des wässrigen organischen Zweiphasensystems.

Gemäß einer weiteren Ausführungsform wird im Verfahren zusätzlich ein organisches Lösungsmittel, das nicht an der Regeneration des Cofaktors beteiligt ist, wie beispielsweise Diethylether, tertiär-Butylmethylether, Diisopropylether, Dibutylether, Ethylacetat, Butylacetat, Heptan, Hexan oder Cyclohexan, eingesetzt.

Bevorzugt ist weiters, dass der TTN des erfindungsgemäßen Verfahrens ≥10³ ist.

Außerdem werden vorzugsweise mindestens 50 % der eingesetzten Oxosteroidverbindung bzw. Hydroxysteroidverbindung innerhalb von 2 bis 96 h zur korrespondierenden Hydroxysteroidverbindung reduziert bzw. zur Oxosteroidverbindung oxidiert.

Bevorzugte Ausführungsformen des erfindungsgemäßen Verfahrens sind dadurch gekennzeichnet, dass als Oxosteroidverbindungen beispielsweise Ketolithocholsäure (Formel II), Dexamethason (Formel III), 4-Androsten-3,17-dion (Formel IV), 1,4-Androstadien-3,17-dion (Formel V), Estron (Formel VI), Pregnenolon (Formel VII) und Cortison (Formel VIII) eingesetzt werden.

Bevorzugte Ausführungsformen des erfindungsgemäßen Verfahrens sind weiterhin dadurch gekennzeichnet, dass als Hydroxysteroidverbindungen beispielsweise unterschiedliche Derivate der Gallensäure wie Cholsäure, Chenodeoxchlosäure, 12-Oxocholsäure, 3-Hydroxy-12-oxocholsäure, Ketolithocholsäure, Lithocholsäure oder auch Hydrocortison eingesetzt werden.

Unter Hydroxysteroiddehydrogenasen werden allgemein solche Enzyme verstanden, die in der Lage sind, die Reduktion von Ketogruppen zu Hydroxygruppen bzw. die Oxidation von Hydroxygruppen zu den entsprechenden Ketogruppen am Steroidgerüst zu katalysieren. Die Oxidation bzw. Reduktion kann dabei am Ringsystem des Steroids selbst (z.B.7-α Hydroxysteroid-dehydrogenasen) oder auch an Kohlenstoffresten des Steroidgrundgerüstes (z.B. 20-β Hydroxysteroiddehydrogenasen) erfolgen.

Geeignete Hydroxysteroiddehydrogenasen zur Reduktion von Oxosteroidverbindungen sind beispielsweise 3-α Hydroxysteroiddehydrogenase (HSDH), 3β HSDH, 12α HSDH, 20β HSDH, 7α HSDH, 7β HSDH, 17β HSDH und 11β HSDH.

Eine geeignete 3-α Hydroxysteroiddehydrogenase ist beispielsweise erhältlich aus *Pseudomonas testosteroni* (J. Biol. Chem. 276 (13), 9961-9970 (2001)) und kann zur Oxidation von 3-α-Hydroxysteroiden bzw. zur Reduktion der 3-Ketosteroide verwendet werden, wie z.B. 3-Keto-Gallensäuren, Progesteron, 4-Androsten-3,17-dion, 5-α-Androstan-3,17-dion etc.

Geeignete Enzyme mit 3-β Hydroxysteroiddehydrogenaseaktivität sind beispielsweise erhältlich aus *Clostridium innocuum* (Applied and Environmental Microbiology, June 1989, p. 1656-1659) oder aus *Pseudomonas testosteroni* und können zur Oxidation von 3-β-Hydroxysteroiden bzw. zur Reduktion der 3-Ketosteroide verwendet werden, wie z.B. 3-Keto-Gallensäuren, Progesteron, 4-Androsten-3,17-dion, 5-α-Androstan-3,17-dion etc.

Eine 12α HSDH ist beispielsweise aus Clostridien erhältlich (Eur. J. Biochem. 196 (1991) 439-450) und kann zur Oxidation von 12α-Hydroxysteroiden (z.B. Cholsäure) bzw. zur Reduktion von 12-Ketosteroiden verwendet werden, wie z.B. von 12-Keto-Gallensäuren (12-Ketochenodeoxycholsäure, Dehydrocholsäure). Ebenso sind Enzyme mit 12-β Hydroxysteroiddehydrogenaseaktivität aus Clostridien beschrieben (Biochim. Biophys. Acta 1988 Oct. 14; 962(3): 362-370).

Enzyme mit 20-β Hydroxysteroiddehydrogenaseaktivität sind beispielsweise erhältlich aus Organismen der Gruppe *Streptomyces* (The Journal of Biological Chemistry, 1977, Vol 252 No 1, Jan 10, 205-211) und können zur Reduktion von Cortison und Cortisolderivaten (Cortison, Cortisol, Cortexolon, Progesteron) zu den entsprechenden 20-β-Hydroxysteroiden verwendet werden (zB. 20-β-Hydroxyprogesteron).

Entsprechende Enzyme mit 20-α Hydroxysteroiddehydrogenaseaktivität sind beispielsweise aus Clostridien, insbesondere aus *Clostridium scindens* (Journal of Bacteriology, June 1989, p. 2925-2932), und aus *Tetrahymena pyriformis* (Biochem.J. (1994) 297, 195-200) erhältlich.

Geeignete 7-α Hydroxysteroiddehydrogenasen sind unter anderem aus Organismen der Darmflora, wie beispielsweise aus Clostridien (*Clostridium absonum, Clostridium sordellii)*(Journal of Bacteriology, Aug. 1994, p. 4865-4874), aus *Escherichia coli* (Journal ofBacteriology Apr., 1991, p. 2173-2179), aus *Bacteroides fragilis* (Current Microbiology, Vol 47 (2003) 475-484), *Brucella, Eubacterium* erhältlich und können zur Oxidation von 7-α-Hydroxysteroiden (Chenolithocholäure) bzw. zur Reduktion von 7-Ketosteroiden verwendet werden, wie z.B. von 7-Keto-Gallensäuren (Ketolithocholsäure).

Entsprechende Enzyme mit 7-β Hydroxysteroiddehydrogenaseaktivität sind ebenfalls beschrieben aus Clostridien, aus Microorganismen der Familie der Ruminococcen (J. Biochemistry 102, 1987, p. 613-619) bzw. Peptostreptococcen (Biochimica and Biophysica Acta 1004, 1989, p. 230-238), aus *Eubacterium aerofaciens* (Applied and Environmental Microbiology, May 1982, p. 1057-1063) und *aus Xanthomonas maltophila* (Pedrini et all, Steroids 71 (2006) p 189-198). Mittels einer 7-β HSDH kann beispielsweise Ursodesoxycholsäure aus Ketolithocholsäure hergestellt werden.

17-β Hydroxysteroiddehydrogenasen sind bekannt aus Pilzen wie *Cylindrocarpon radicola* (J. Biochemistry 103, 1988, 1039-1044) und *Cochliobolus lunatus* (J. Steroid Biochem. Molec.Biol. Vol 59, 1996, No. 2, p. 205-214), aus Bakterien der Familie Streptomyces (Hoppe-Seyler's Z. Physiol. Chem, Bd. 356, 1975, 1843-1852), Pseudomonas (The Journal of Biological Chemistry, Vol. 252 No.11, June 10, 1977, p. 3775-3783) und Alcaligenes (The Journal of Biological Chemistry, Vol. 260, No. 25, Nov 5, 1985, p. 13648-13655).

17-β Hydroxysteroiddehydrogenasen können beispielsweise zur Oxidation von 17-β-Hydroxysteroiden bzw. zur Reduktion von 17-Ketosteroiden verwendet werden, wie z.B. von 4-Androsten-3,17-dione, Androsteron, Estron.

Ein entsprechendes Enzym mit 17-α Hydroxysteroiddehydrogenaseaktivität ist beschrieben aus *Eubacterium* sp. (Journal of Lipid Research, Vol. 35, 1994, p. 922-929).

11-β Hydroxysteroiddehydrogenasen sind bekannt aus höheren Säugetieren und können beispielsweise verwendet werden, um Cortisol zu Cortison zu oxidieren.

Es kann aber auch jede andere Oxidoreduktase als Hydroxysteroiddehydrogenase verwendet werden, die Oxidationen bzw. Reduktionen am Steroidgerüst katalysiert.

Geeignete sekundäre Alkoholdehydrogenasen zur Regenerierung des NADH bzw. NAD, beispielsweise bei Verwendung der 17-β Hydroxysteroiddehydrogenasen aus *Pseudomonas testosteroni,* der 3-β Hydroxysteroiddehydrogenasen aus *Clostridium innocuum* oder der 7-α Hydroxysteroiddehydrogenase aus *Bacteroides fragilis,* sind z.B. solche wie oben beschrieben und werden isoliert aus Hefen der Gattungen Candida und Pichia, wie z.B.: Carbonylreduktase aus *Candida parapsilosis* (CPCR) (US 5,523,223 und US 5,763,236;
Enzyme Microb. Technol. 1993 Nov; 15(11):950-8),
*Pichia capsulata* (DE 10327454.4), *Pichia farinosa* (A 1261/2005, Kl. C12N), *Pichia finlandica* (EP 1179595 A1), *Candida nemodendra* (A 1261/2005, Kl. C12N), *Pichia trehalophila* (A 1261/2005, Kl. C12N), *Rhodotorula mucilaginosa* (A 1261/2005, Kl. C12N), *Lodderomyces elongisporus* (A 1261/2005, Kl. C12N) und *Pichia stipidis* (A 1261/2005, Kl. C12N)

Des weiteren kann die Regenerierung des NADH auch mit sekundären Alkoholdehydrogenasen / Oxidoreduktase, wie beschrieben und isoliert aus Bakterien der Klasse der Actinobacteria, z.B. aus *Rhodococcus erythropolis* (US 5,523,223), *Norcardia , fusca* (Biosci. Biotechnol. Biochem., 63 (10) (1999), Seiten 1721-1729; Appl. Microbiol. Biotechnol. 2003 Sep; 62(4):380-6, Epub 2003 Apr 26), *Rhodococcus ruber* (J. Org. Chem. 2003 Jan 24; 8(2):402-6.) oder *Microbacterium spec.* (A 1261/2005, Kl. C12N), erfolgen.

Geeignete sekundäre Alkoholdehydrogenasen / Oxidoreduktasen zur Regenerierung des NADPH bzw. NADP, beispielsweise bei Verwendung der 12-α Hydroxysteroiddehydrogenasen aus *Clostridium paraputrificum,* der 17-α, Hydroxysteroiddehydrogenasen aus *Eubacterium* sp. oder der 7-α Hydroxysteroiddehydrogenase aus *Clostridium sordelli,* sind beispielsweise solche, wie beschrieben und isoliert aus Organismen der Ordnung Lactobacillales *(Lactobacillus kefir* (US 5,200,335), *Lactobacillus brevis* (DE 19610984 A1; Acta Crystallogr. D. Biol. Crystallogr. 2000 Dec; 56 Pt 12:1696-8), *Lactobacillus minor* (DE 10119274), *Leuconostoc carnosum* (A 1261/2005, Kl. C12N) oder solche, wie beschrieben, aus *Thermoanerobium brockii, Thermoanerobium ethanolicus* oder *Clostridium beijerinckii.*

Beide Enzyme, Hydroxysteroiddehydrogenase und Oxidoreduktase / Alkoholdehydrogenase, werden bevorzugt rekombinant überexprimiert in *Escherichia coli* verwendet. Im erfindungsgemäßen Verfahren können beide Enzyme, Hydroxysteroiddehydrogenase und Alkoholdehydrogenase / Oxidoreduktase, entweder vollständig gereinigt, teilweise gereinigt oder in Zellen enthalten eingesetzt werden. Die eingesetzten Zellen können dabei nativ, permeabilisiert oder lysiert vorliegen.

Je kg umzusetzender Oxosteroidverbindung bzw. Hydroxysteroidverbindung werden 50 000 bis 10 Mio U Hydroxysteroiddehydrogenase und 50 000 bis 10 Mio U Alkoholdehydrogenase eingesetzt (nach oben offen).

Der Enzymeinheit 1 U entspricht dabei diejenige Enzymmenge an Hydroxysteroiddehydrogenase, die benötigt wird, um 1 µmol Oxosteroidverbindung pro Minute (min) umzusetzen, bzw. der Enzymmenge an Alkoholdehydrogenase, die benötigt wird, um 1 µmol 2-Alkohol pro Minute (min) zu oxidieren.

Analog kann je kg umzusetzender Oxosteroidverbindung bzw. Hydroxysteroidverbindung ca. 10 g bis 500 g Biofeuchtmasse von E.coli, enthaltend die Hydroxysteroiddehydrogenase, und 10 g bis 500 g Biofeuchtmasse von E.coli, enthaltend die Alkoholdehydrogenase /Oxidoreduktase, eingesetzt (nach oben offen).

Die Regenerierung von NAD(P)H erfolgt im beschriebenen Verfahren enzymgekoppelt.

Die Umsetzung der Oxosteroidverbindung bzw. der Hydroxysteroidverbindung erfolgt im erfindungsgemäßen Verfahren bevorzugt im Zweiphasensystem, enthaltend einen 2-Alkohol bzw. eine Ketoverbindung zur Cofactorregenerierung, eine Hydroxysteroiddehydrogenase, eine Alkoholdehydrogenase, Wasser, Cofaktor und die Oxosteroidverbindung bzw. Hydroxysteroidverbindung. Weiters können noch zusätzliche organische Lösungsmittel enthalten sein, die nicht an der Cofactorregenerierung beteiligt sind, d.h. keine durch die verwendete Alkoholdehydrogenase oxidierbare Hydroxygruppen bzw. reduzierbare Ketogruppe enthalten.

Der Anteil der nicht wassermischbaren organischen Komponenten des Zweiphasensystems kann dabei zwischen 10% und 90%, bevorzugt von 20% bis 80%, bezogen auf das Gesamtvolumen des Reaktionsansatzes betragen. Der wässrige Anteil kann von 90% bis 10%, bevorzugt von 80% bis 20%, bezogen auf das Gesamtvolumen des Reaktionsansatzes, betragen.

Dem Wasser kann ein Puffer zugesetzt werden, beispielsweise Kaliumphosphat-, Tris/HCl-, Glycin- oder Triethanolamin-Puffer mit einem pH-Wert von 5 bis 10, vorzugsweise von 6 bis 9. Der Puffer kann zusätzlich noch Ionen zur Stabilisierung oder Aktivierung beider Enzyme enthalten, beispielsweise Magnesiumionen oder Zinkionen.

Des weiteren können im erfindungsgemäßen Verfahren noch weitere Zusätze zur Stabilisierung der Enzyme Hydroxysteroiddehydrogenase und Alkoholdehydrogenase eingesetzt werden, wie beispielsweise Glycerin, Sorbitol, 1,4-DL-Dithiothreit (DTT) oder Dimethylsulfoxid (DMSO).

Die Konzentration des Cofaktors NAD(P)H, bezogen auf die wässrige Phase, beträgt von 0,001 mM bis 10 mM, insbesondere von 0,01 mM bis 1,0 mM. Die Temperatur kann in Abhängigkeit von den speziellen Eigenschaften der eingesetzten Enzyme von 10°C bis 70°C betragen, bevorzugt von 20°C bis 35°C.

Der im erfindungsgemäßen Verfahren erreichten TTN (total turn over number = mol reduzierte Oxosteroidverbindung / mol eingesetzter-Cofactor) kann dabei im Bereich von 10² bis 10⁵ liegen, in der Regel wird ein TTN >10³ bevorzugt.

Die zu reduzierenden Oxosteroidverbindungen bzw. Hydroxysteroidverbindungen sind in der Regel schwer wasserlöslich. Das Substrat kann während der Reaktion vollständig oder auch unvollständig gelöst vorliegen. Ist das Substrat im Reaktionsgemisch nicht vollständig gelöst, so liegt ein Teil des Substrates in fester Form vor und kann so eine dritte feste Phase bilden. Das Reaktionsgemisch kann während der Umsetzung auch zeitweise eine Emulsion bilden.

Die zu reduzierende Oxosteroidverbindung bzw. zu oxidierende Hydroxysteroidverbindung wird im erfindungsgemäßen Verfahren in Mengen ≥ 50 g/l, bezogen auf das Gesamtvolumen des Reaktionsansatzes, eingesetzt. Bevorzugt werden zwischen 50g/l und 400g/l Oxosteroidverbindung / Hydroxysteroidverbindung, besonders bevorzugt zwischen 50g/l und 200g/l, eingesetzt.

Die bevorzugten zusätzlichen organischen Lösungsmittel, die nicht an der Regenerierung des Cofactors beteiligt sind, sind beispielsweise Ethylacetat, Butylacetat, tertiär-Butylmethylether, Diisopropylether, Heptan, Hexan, Toluol, Dichlormethan oder Cyclohexan oder deren Gemische unterschiedlicher Zusammensetzung.

Die Regeneration des NAD(P)H wird durch Oxidation sekundärer Alkohole der allgemeinen Formel R_{X}R_{Y}CHOH bzw. C4-C6-Cycloalkanole erreicht. Als Reaktionsprodukte entstehen dabei Ketone der allgemeinen Formel R_{X}R_{Y}C=O bzw. C4-C6-Cycloalkanone. Bevorzugte sekundäre Alkohole sind dabei aliphatische 2-Alkohole, wie beispielsweise Isopropanol, 2-Butanol, 2-Pentanol, 2-Hexanol, 2-Heptanol, 2-Oktanol, 4-Methyl-2-pentanol , 5 Methyl-2-hexanol, und cyclische sekundäre Alkohole, wie Cyclohexanol, Cyclopentanol.

Die Regeneration des NAD(P) wird durch Reduktion von Ketoverbindungen der allgemeinen Formel R_{X}R_{Y}C=O bzw. von C4-C6-Cycloalkanonen erreicht. Als Reaktionsprodukte entstehen dabei sekundäre Alkohole der allgemeinen Formel R_{X}R_{Y}CHOH bzw. C4-C6-Cycloalkanole. Bevorzugte Ketoverbindungen sind dabei Ketone, wie beispielsweise Aceton, 2-Butanon, 2-Pentanon, 2-Hexanon, 2-Heptanon, 2-Oktanon, 4-Methyl-2-pentanon , 5 Methyl-2-hexanon, und cyclische Ketone, wie Cyclohexanon, Cyclopentanon.

Das erfindungsgemäße Verfahren wird beispielsweise in einem Reaktionsgefäß aus Glas oder Metall durchgeführt. Dazu werden die Komponenten einzeln in das Reaktionsgefäß überführt und unter einer Atmosphäre von beispielsweise Stickstoff oder Luft gerührt. Je nach eingesetzter Oxosteroidverbindung beträgt die Reaktionszeit von 1 Stunde bis 96 Stunden, insbesondere von 2 Stunden bis 48 Stunden. In dieser Zeit wird die Oxosteroidverbindung zu mindestens 50 % zum korrespondierenden Hydroxysteroid reduziert bzw. das Hydroxysteroid zur Oxosteroidverbindung oxidiert.

Die vorliegende Erfindung wird nachfolgend anhand von Beispielen näher erläutert.

### Beispiele:

### 1. Reduktion von Androsten-3,17-dion zu 17-β-Hydroxy-4-androsten-3-on (Testosteron)

### A) Zweiphasensystem mit 4-Methyl-2-pentanol zur Coenzymregenerierung

Zur Synthese von 17 β-Hydroxy-4-androsten-3-on (Testosteron) werden zu 0,5 ml Puffer (100 mM Triethanolamin, pH = 7, 1 mM MgCl₂, 10% Glycerin) enthaltend 0,1 mg NAD, 30 Units der rekombinanten 17-β- Hydroxysteroiddehydrogenase aus *Pseudomonas testosteroni* ( J. Steroid Biochem. Mol. Biol. 44 (2), 133-139 (1993), Pubmed P19871) und 50 Units der rekombinanten Alkoholdehydrogenase aus *Pichia capsulata* (DE-A 103 27 454) 100 mg Androsten-3,17-dione, gelöst in 0,4 ml 4-Methyl-2-pentanol, zugegeben. Das Gemisch wird 24 h bei Raumtemperatur und unter ständiger Durchmischung inkubiert. Die Konzentration an Androsten-3,17-dion am Gesamtreaktionsvolumen beträgt ca. 100g/l.

Nach Beendigung der Reaktion kann das Reaktionsgemisch beispielsweise aufgearbeitet werden, indem das Reaktionsgemisch mit einem organischem Lösungsmittel extrahiert wird und das Lösungsmittel anschließend mittels Destillation entfernt wird.

Nach 24 h sind ca. 94 % des eingesetzten Androsten-3,17-dion zu 17-β-Hydroxy-4-androsten-3-on (Testosteron) umgesetzt.

Die Umsetzung des Androsten-3,17-dion zu 17-β-Hydroxy-4-androsten-3-on (Testosteron) wurde mittels Gaschromatographie verfolgt. Hierzu wurde ein Gaschromatograph GC-17A von Shimadzu mit einer chiralen Trennsäule Lipodex E, 12m (Machery-Nagel, Düren, Deutschland), Flammen-Ionisations-Detektor und Helium als Trägergas benutzt.

### B) Zweiphasensystem mit Butylacetat und 2-Propanol zur Coenzymregenerierung

Zur Synthese von 17-β-Hydroxy-4-androsten-3-on (Testosteron) werden zu 0,5 ml Puffer (100 mM Triethanolamin, pH = 7, 1 mM MgCl₂, 10% Glycerin), enthaltend 0,1 mg NAD, 30 Units der rekombinanten 17-β-Hydroxysteroiddehydrogenase aus *Pseudomonas testosteroni* (J. Steroid Biochem. Mol. Biol. 44 (2), 133-139 (1993), Pubmed P19871) und 50 Units der rekombinanten Alkoholdehydrogenase aus *Pichia capsulata* (DE-A 103 27 454) 100 mg Androsten-3,17-dion, gelöst in 0,3 ml Butylacetat und 0,1 ml 2-Propanol, zugegeben. Das Gemisch wird 24 h bei Raumtemperatur und unter ständiger Durchmischung inkubiert. Die Konzentration an Androsten-3,17-dion am Gesamtreaktionsvolumen beträgt ca. 100g/l.

Nach Beendigung der Reaktion kann das Reaktionsgemisch beispielsweise aufgearbeitet werden, indem das Reaktionsgemisch mit einem organischem Lösungsmittel extrahiert wird und das Lösungsmittel anschließend mittels Destillation entfernt wird.

Nach 24 h sind ca. 90-95% des eingesetzten Androsten-3,17-dion zu 17-β-Hydroxy-4-androsten-3-on (Testosteron) umgesetzt.

### 2. Reduktion von Ketolithocholsäure zu Chenolithocholsäure

### A) Coenzymrepeneration mit ADH aus Thermoanerobium brockii / Zweiphasensystem

Zur Synthese von 3α-7α-Dihydroxy-5-β-cholansäure (Chenodeoxycholsäure) werden zu 0,2 ml Puffer (100 mM Kaliumphosphatpuffer, pH = 8,5, 1 mM MgCl₂, 10% Glycerin), enthaltend 0,1 mg NADP, 10 Units der rekombinanten 7-α-Hydroxysteroiddehydrogenase aus *Clostridiem scindens* (Pubmed AAB61151) und 10 Units der rekombinanten Alkoholdehydrogenase aus *Thermoanerobium brockii ,* 100 mg 3α-Hydroxy-7-oxo-5-β-cholansäure (Ketolithocholsäure) in 0,5 ml Methylpentanol zugegeben. Das Gemisch wird 24 h bei Raumtemperatur und unter ständiger Durchmischung inkubiert. Die Konzentration an Ketolithocholsäure am Gesamtreaktionsvolumen beträgt ca. 100g/l.

Nach 24 h sind ca. 90-98% der eingesetzten Ketolithocholsäure zu Chenodeoxycholsäure umgesetzt.

Die Umsetzung von Ketolithocholsäure zu Chenodeoxycholsäure wurde mittels HPLC verfolgt. Hierzu wurde eine Trennsäule EC125/4 Nucleodur 100-5 C18ec (Machery-Nagel, Düren, Deutschland) isokratisch mit einem Gemisch aus MeOH/Wasser (80:20) verwendet.

B) Coenzymreizeneration mit ADH aus Lactobacillus (DE 10119274) / Zweiphasensystem

Zur Synthese von 3α-7α-Dihydroxy-5-β-cholansäure (Chenodeoxycholsäure) werden beispielsweise zu 0,3 ml Puffer (100 mM Triethanolaminpuffer, pH = 7, 1 mM MgCl₂, 10% Glycerin), enthaltend 0,1 mg NADP, 10 Units der rekombinanten 7-α-Hydroxysteroiddehydrogenase aus *Clostridium scindens* (Pubmed AAB61151) und 10 Units der rekombinanten Alkoholdehydrogenase aus Lactobacillus (DE 10119274), 100 mg 3α-Hydroxy-7-oxo-5-β-cholansäure (Ketolithocholsäure) in 0,5 ml Oktanol zugegeben. Das Gemisch wird 24 h bei Raumtemperatur und unter ständiger Durchmischung inkubiert. Die Konzentration an Ketolithocholsäure am Gesamtreaktionsvolumen beträgt ca. 100g/l.

Nach 24 h sind ca. 70-80% der eingesetzten Ketolithocholsäure zu Chenodeoxycholsäure umgesetzt.

### 3. Reduktion von 1,4-Androstadien-3,17 dion zu 17-β-Hydroxy-1,4-androstadien-3-on

Zur Synthese von 17-β-Hydroxy-1,4-androstadien-3-on werden zu 0,5 ml Puffer (100 mM Triethanolamin, pH = 7, 1 mM MgCl₂, 10% Glycerin), enthaltend 0,1 mg NAD, 30 Units der rekombinanten 17-β-Hydroxysteroiddehydrogenase aus *Pseudomonas testosteroni* (J. Steroid Biochem. Mol. Biol. 44 (2), 133-139 (1993), Pubmed P19871) und 5 Units der rekombinanten Alkoholdehydrogenase aus *Pichia capsulata* (DE-A 103 27 454), 100 mg 1,4-Androstadien-3,17-dion, gelöst in 0,4 ml 4-Methyl-2-pentanol, zugegeben. Das Gemisch wird 24 h bei Raumtemperatur und unter ständiger Durchmischung inkubiert. Die Konzentration an 1,4-Androstadiene-3,17-dion am Gesamtreaktionsvolumen beträgt ca. 100g/l.

Nach Beendigung der Reaktion kann das Reaktionsgemisch beispielsweise aufgearbeitet werden, indem die organische Phase abgetrennt und das Lösungsmittel anschließend mittels Destillation entfernt wird.

Nach 24 h sind ca. 90-98% des eingesetzten Androsten-3,17-dion zu 17-β-Hydroxy-1,4-androstadien-3-on umgesetzt.

Die Umsetzung des 1,4-Androstadien-3,17-dion zu 17-(3-Hydroxy-1,4-androstadien-3-on wurde mittels Gaschromatographie verfolgt. Hierzu wurde ein Gaschromatograph Autosystem XL von Perkin Elmer ausgerüstet mit Massenspektometer mit einer FS-Kapillarsäule Optima -5-MS (Machery-Nagel, Düren, Deutschland) und Helium als Trägergas verwendet.

### 4. Oxidation von Chenolithocholsäure zu Ketolithocholsäure

### A) Coenzymregeneration mit ADH aus Thermoanerobium brockii / Zweiphasensystem

Zur Synthese von 3α-Hydroxy-7-oxo-5-β-cholansäure (Ketolithocholsäure) werden zu 0,4 ml Puffer (100 mM Kaliumphosphatpuffer, pH = 8,5, 1 mM MgCl₂, 10% Glycerin), enthaltend 0,1 mg NADP, 10 mg Biofeuchtmasse E.coli, enthaltend die überexprimierte 7-α-Hydroxysteroiddehydrogenase aus *Clostridiem scindens* (Pubmed AAB61151), und 5 mg Biofeuchtmasse E.coli, enthaltend die überexprimierte Alkoholdehydrogenase aus *Thermoanerobium brockii,* 100 mg 3α-7α-Dihydroxy-5-β-cholansäure (Chenodeoxycholsäure) in 0,5 ml Methylisobutylketon zugegeben. Das Gemisch wird 24 h bei Raumtemperatur und unter ständiger Durchmischung inkubiert. Die Konzentration an Ketolithocholsäure am Gesamtreaktionsvolumen beträgt ca. 100g/l.

Nach 24 h sind mehr als 80 % der eingesetzten Chenodeoxycholsäure zu Ketolithocholsäure umgesetzt.

Die Umsetzung von Ketolithocholsäure zu Chenodeoxycholsäure wurde mittels Dünnschichtchromatographie verfolgt.

B) Coenzymregeneration mit ADH aus Lactobacillus (DE 10119274) / Zweiphasensystem

Zur Synthese von 3α-Hydroxy-7-oxo-5-β-cholansäure (Ketolithocholsäure) werden zu 0,15 ml Puffer (100 mM Kaliumphosphatpuffer, pH = 7,5, 1 mM MgCl₂, 10% Glycerin), enthaltend 0,1 mg NADP, 10 mg Biofeuchtmasse E.coli, enthaltend die überexprimierte 7-α-Hydroxysteroiddehydrogenase aus *Clostridiem scindens* (Pubmed AAB61151), und 5 mg Biofeuchtmasse E.coli, enthaltend die überexprimierte Alkoholdehydrogenase aus Lactobacillus (DE 10119274), 100 mg 3α-7α-Dihydroxy-5-β-cholansäure (Chenodeoxycholsäure) in 0,7 ml Methylisobutylketon zugegeben. Das Gemisch wird 24 h bei Raumtemperatur und unter ständiger Durchmischung inkubiert. Die Konzentration an Ketolithocholsäure am Gesamtreaktionsvolumen beträgt ca. 100g/l.

Nach 24 h sind mehr als 90 % der eingesetzten Chenodeoxycholsäure zu Ketolithocholsäure umgesetzt.

## Patentansprüche

1. Verfahren zur enantioselektiven enzymatischen Reduktion von Oxosteroidverbindungen der allgemeinen Formel I 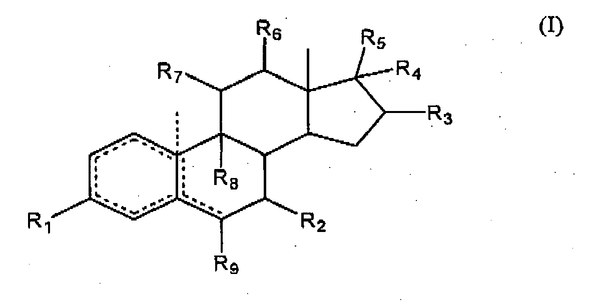 worin
R₁ Wasserstoff, eine Methylgruppe, eine Hydroxygruppe oder eine Oxogruppe,
R₂ Wasserstoff, eine Methylgruppe, eine Hydroxygruppe oder eine Oxogruppe,
R₃ Wasserstoff, eine Hydroxygruppe, eine Oxogruppe oder eine Methylgruppe,
R₄ Wasserstoff oder eine Hydroxygruppe,
R₅ Wasserstoff, ein Rest -COR₁₀, wobei R₁₀ eine unsubstituierte oder mit einer Hydroxygruppe substituierte C1-C4-Alkylgruppe ist, oder eine substituierte oder unsubstituierte C1-C4-Carboxyalkylgruppe,
oder R₄ und R₅ zusammen eine Oxogruppe,
R₆ Wasserstoff, eine Methylgruppe, eine Hydroxygruppe oder eine Oxogruppe,
R₇ Wasserstoff eine Methylgruppe, eine Hydroxygruppe oder eine Oxogruppe,
R₈ Wasserstoff eine Methylgruppe oder ein Halogenid, und
R₉ Wasserstoff, eine Methylgruppe, eine Hydroxygruppe, eine Oxogruppe oder ein Halogenid repräsentieren,
wobei mindestens einer von R₁, R₂, R₄+R₅, R₆, R₈ oder R₉ eine Oxogruppe bzw. R₅ ein Rest -COR₁₀ ist und das Strukturelement 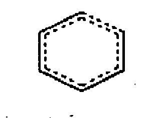
einen Benzolring oder einen C6-Ring mit 0, 1 oder 2 C-C-Doppelbindungen repräsentiert,
oder der Formel II (Ketolithocholsäure) 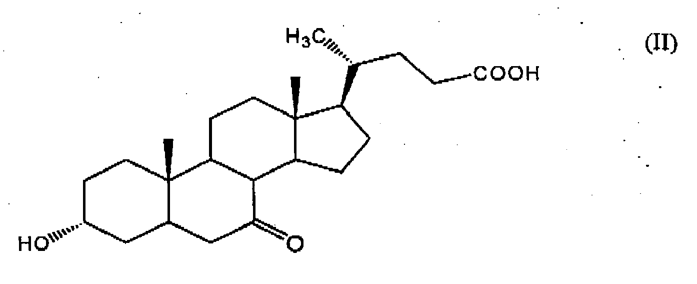 wobei die Oxosteroidverbindung mit einer Hydroxysteroiddehydrogenase in Gegenwart eines Cofactors NADH oder NADPH reduziert wird, **dadurch gekennzeichnet, dass**
a) die Oxosteroidverbindung in einer Konzentration von ≥50 g/l im Reaktionsansatz vorliegt,
b) der durch die Hydroxysteroiddehydrogenase gebildete oxidierte Cofactor NAD oder NADP kontinuierlich durch Oxidation eines sekundären Alkohols der allgemeinen Formel R_{X}R_{Y}CHOH, wobei R_{X}, R_{Y} unabhängig voneinander verzweigtes oder unverzweigtes Cl-C8-Alkyl darstellen und C_{insgesamt} ≥ 3, oder durch Oxidation eines C4-C6-Cycloalkanols regeneriert wird, und
c) zur Oxidation des sekundären Alkohols der allgemeinen Formel R_{X}R_{Y}CHOH bzw. des Cycloalkanols eine zusätzliche Oxidoreduktase/Alkoholdehydrogenase eingesetzt wird.

2. Verfahren nach Anspruch 1, **dadurch gekennzeichnet, dass** als sekundärer Alkohol der allgemeinen Formel R_{X}R_{Y}CHOH 2-Propanol, 2-Butanol, 2-Pentanol, 4-Methyl-2-pentanol, 2-Hexanol, 2-Heptanol, 5-Methyl-2-hexanol oder 2-Octanol und als Cycloalkohol Cyclohexanol eingesetzt wird.

3. Verfahren nach Anspruch 1 oder 2, **dadurch gekennzeichnet, dass** es in einem wässrigen organischen Zweiphasensystem durchgeführt wird.

4. Verfahren nach Anspruch 3, **dadurch gekennzeichnet, dass** zusätzlich ein organisches Lösungsmittel, wie beispielsweise Diethylether, tertiär-Butylmethylether, Diisopropylether, Dibutylether, Ethylacetat, Butylacetat, Heptan, Hexan oder Cyclohexan, eingesetzt wird.

5. Verfahren nach einem der Ansprüche 1 bis 4, **dadurch gekennzeichnet, dass** der TTN, d.h. total turn over number = mol reduziertes Oxosteroidverbindung/ mol eingesetzter Cofactor ≥10³ ist.

6. Verfahren nach einem der Ansprüche 1 bis 5, **dadurch gekennzeichnet, dass** innerhalb von 2 bis 96 h mindestens 50 % der eingesetzten Oxosteroidverbindung zur Hydroxysteroidverbindung reduziert werden.

7. Verfahren nach einem der Ansprüche 1 bis 6, **dadurch gekennzeichnet, dass** als Oxosteroidverbindung Dexamethason (Formel III) eingesetzt wird.

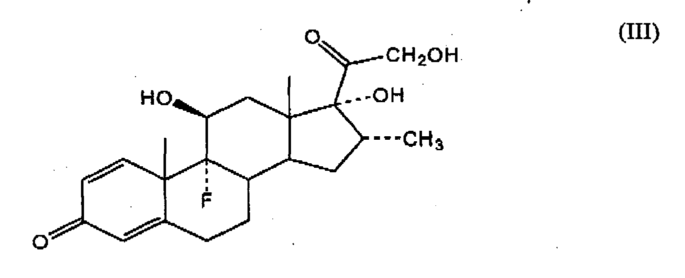

8. Verfahren nach einem der Ansprüche 1 bis 6, **dadurch gekennzeichnet, dass** als Oxosteroidverbindung 4-Androsten-3,17-dion (Formel IV) eingesetzt wird.

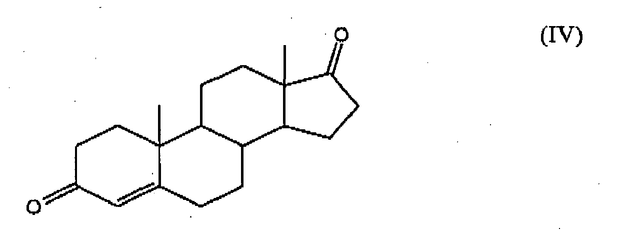

9. Verfahren nach einem der Ansprüche 1 bis 6, **dadurch gekennzeichnet, dass** als Oxosteroidverbindung 1,4-Androstadien-3,17-dion (Formel V) eingesetzt wird.

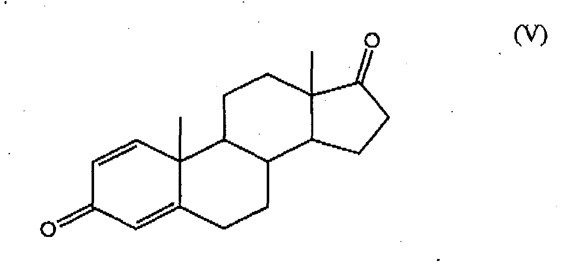

10. Verfahren nach einem der Ansprüche 1 bis 6, **dadurch gekennzeichnet, dass** als Oxosteroidverbindung Estron (Formel VI) eingesetzt wird.

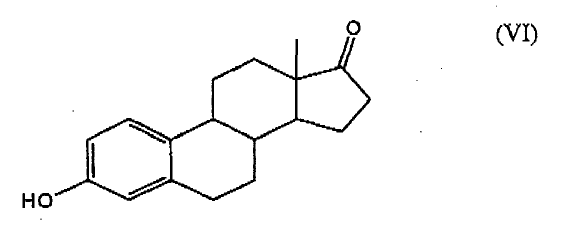

11. Verfahren nach einem der Ansprüche 1 bis 6, **dadurch gekennzeichnet, dass** als Oxosteroidverbindung Pregnenolon (Formel VII) eingesetzt wird.

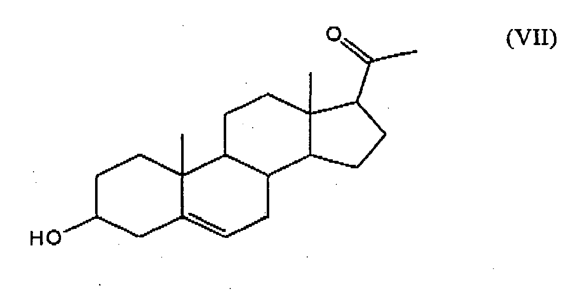

12. Verfahren nach einem der Ansprüche 1 bis 6, **dadurch gekennzeichnet, dass** als Oxosteroidverbindung Cortison (Formel VIII) eingesetzt wird.

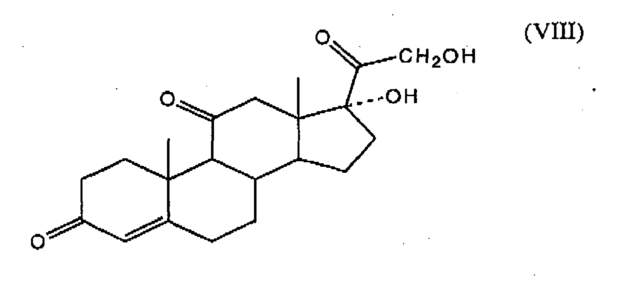

13. Verfahren zur enzymatischen Oxidation von Hydroxysteroidverbindungen der allgemeinen Formel I 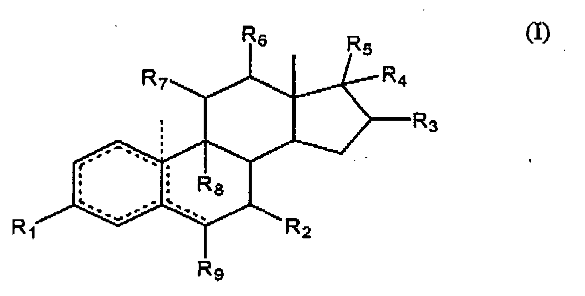 worin
R₁ Wasserstoff, eine Methylgruppe, eine Hydroxygruppe oder eine Oxogruppe,
R₂ Wasserstoff, eine Methylgruppe, eine Oxogruppe oder eine Hydroxygruppe,
R₃ Wasserstoff, eine Hydroxygruppe, eine Oxogruppe oder eine Methylgruppe,
R₄ Wasserstoff oder eine Hydroxygruppe,
R₅ Wasserstoff, ein Rest -COR₁₀, wobei R₁₀ eine unsubstituierte oder mit einer Hydroxygruppe substituierte C1-C4-Alkylgruppe ist, oder eine substituierte oder unsubstituierte C1-C4-Carboxyalkylgruppe,
oder R₄ und R₅ zusammen eine Oxogruppe,
R₆ Wasserstoff, eine Methylgruppe, eine Oxogruppe oder eine Hydroxygruppe,
R₇ Wasserstoff, eine Methylgruppe, eine Oxogruppe oder eine Hydroxygruppe,
R₈ Wasserstoff, eine Methylgruppe oder ein Halogenid, und
R₉ Wasserstoff, eine Methylgruppe, eine Hydroxygruppe, eine Oxogruppe oder ein Halogenid repräsentieren,
wobei mindestens einer von R₁, R₂, R₄, R₆, R₇, R₈ oder R₉ eine Hydroxygruppe ist und das Strukturelement 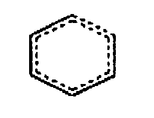
einen Benzolring oder einen,C6-Ring mit 0, 1 oder 2 C-C-Doppelbindungen repräsentiert, oder von den Gallensgurederivaten Cholsäure, Chenodeoxycholsäure, 12-Oxocholsäure, 3-Hydroxy-12-oxocholsäure, Ketolithocholsäure und Litocholsäure,
wobei die Hydroxysteroidverbindung mit einer Hydroxysteroiddehydrogenase in Gegenwart eines Cofactors NAD oder NADP oxidiert wird, **dadurch gekennzeichnet, dass**
a) die Hydroxysteroidverbindung in einer Konzentration von ≥50 g/l im Reaktionsansatz vorliegt,
b) der durch die Hydroxysteroiddehydrogenase gebildete reduzierte Cofactor NADH oder NADPH kontinuierlich durch Reduktion einer Ketoverbindung der allgemeinen Formel R_{X}R_{Y}CO, wobei R_{X}, R_{Y} unabhängig voneinander verzweigtes oder unverzweigtes C1-C8-Alkyl darstellen und C_{insgesamt} ≥ 3, oder durch Reduktion eines C4-C6-Cycloalkanons regeneriert wird, tmd
c) zur Reduktion der Ketoverbindung der allgemeinen Formel R_{X}R_{Y}CO bzw. des Cycloalkanons eine zusätzliche Oxidoreduktase/Alkoholdehydrogenase eingesetzt wird.

14. Verfahren nach Anspruch 13, **dadurch gekennzeichnet, dass** als Keton der allgemeinen Formel R_{X}R_{Y}CO Aceton, 2-Butanon, 2-Pentanon, 4-Methyl-2-pentanon, 2-Hexanon, 2-Heptanon, 5-Methyl-2-hexanon oder 2-Octanon und als Cycloalkanon Cyclohexanon eingesetzt wird.

15. Verfahren nach Anspruch 13 oder 14, **dadurch gekennzeichnet, dass** es in einem wässrigen organischen Zweiphasensystem durchgeführt wird.

16. Verfahren nach Anspruch 15, **dadurch gekennzeichnet, dass** zusätzlich ein organisches Lösungsmittel, wie beispielsweise Diethylether, tertiär-Butylmethylether, Diisopropylether, Dibutylether, Ethylacetat, Butylacetat, Heptan, Hexan oder Cyclohexan, einsetzt wird.

17. Verfahren nach einem der Ansprüche 13 bis 16, **dadurch gekennzeichnet, dass** der TTN i d.n. total turn over number = mol oxidierter Hydroxysteroidverbindung/ mol eingesetzten Cofactor ≥10³ ist.

18. Verfahren nach einem der Ansprüche 13 bis 17, **dadurch gekennzeichnet, dass** innerhalb von 2 bis 96 h mindestens 50 % der eingesetzten Hydroxysteroidverbindung zur Oxosteroidverbindung oxidiert werden.

## Claims

1. A process for the enantioselective enzymatic reduction of oxosteroid compounds of general formula I 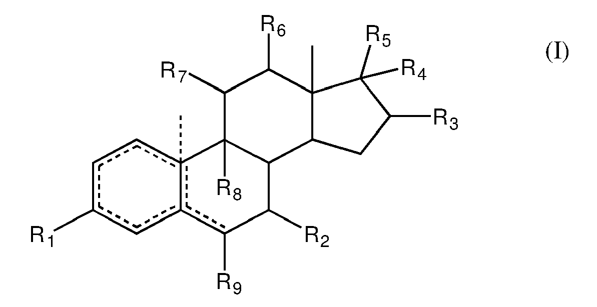 wherein
R₁ represents hydrogen, a methyl group, a hydroxy group or an oxo group,
R₂ represents hydrogen, a methyl group, a hydroxy group or an oxo group,
R₃ represents hydrogen, a hydroxy group, an oxo group or a methyl group,
R₄ represents hydrogen or a hydroxy group,
R₅ represents hydrogen, a moiety -COR₁₀, wherein R₁₀ is a C1-C4-alkyl group that is unsubstituted or substituted with a hydroxy group or a substituted or unsubstituted C1-C4-carboxyalkyl group,
or R₄ and R₅ together represent an oxo group,
R₆ represents hydrogen, a methyl group, a hydroxy group or an oxo group,
R₇ represents hydrogen, a methyl group, a hydroxy group or an oxo group,
R₈ represents hydrogen, a methyl group or a halide, and
R₉ represents hydrogen, a methyl group, a hydroxy group, an oxo group or a halide, wherein at least one of R₁, R₂, R₄+R₅, R₆, R₈ or R₉ is an oxo group, or R₅ is a moiety -COR₁₀, respectively,
and the structural element 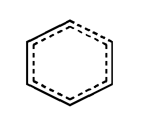
represents a benzene ring or a C6-ring having 0, 1 or 2 C-C-double bonds;
or of formula II (ketolithocholic acid) 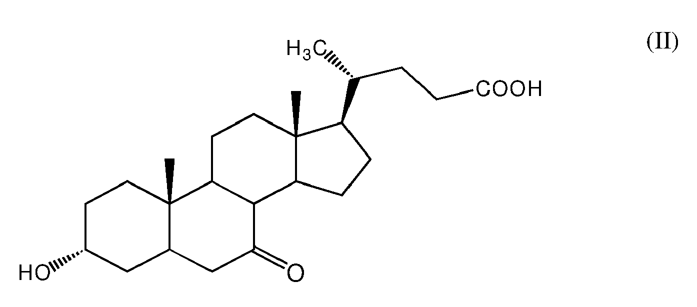 wherein the oxosteroid compound is reduced with a hydroxysteroid dehydrogenase in the presence of a cofactor NADH or NADPH, **characterized in that**
a) the oxosteroid compound is provided in the reaction at a concentration of ≥ 50 g/l,
b) the oxidized cofactor NAD or NADP formed by the hydroxysteroid dehydrogenase is regenerated continuously by oxidation of a secondary alcohol of general formula R_{X}R_{Y}CHOH, wherein Rₓ, R_{Y} independently represent a branched or unbranched C1-C8-alkyl and Cₜₒₜₐₗ≥ 3, or by oxidation of a C4-C6-cycloalkanol, and
c) an additional oxidoreductase/alcohol dehydrogenase is used for the oxidation of the secondary alcohol of general formula R_{X}R_{Y}CHOH or of the cycloalkanol, respectively.

2. The process according to claim 1, **characterized in that** 2-propanol, 2-butanol, 2-pentanol, 4-methyl-2-pentanol, 2-hexanol, 2-heptanol, 5-methyl-2-hexanol or 2-octanol is used as the secondary alcohol of general formula R_{X}R_{Y}CHOH, and cyclohexanol is used as the cycloalcohol.

3. The process according to claim 1 or 2, **characterized in that** it is carried out in an aqueous organic two-phase system.

4. The process according to claim 3, **characterized in that** an organic solvent such as, for example, diethyl ether, tertiary butyl methyl ether, diisopropyl ether, dibutyl ether, ethyl acetate, butyl acetate, heptane, hexane or cyclohexane is additionally employed.

5. The process according to any of claims 1 to 4, **characterized in that** the TTN, i.e., total turn over number = mol of reduced oxosteroid compound / mol of cofactor used, is ≥10³.

6. The process according to any of claims 1 to 5, **characterized in that** at least 50% of the employed oxosteroid compound is reduced to the hydroxysteroid compound within 2 to 96 h.

7. A process according to any of claims 1 to 6, **characterized in that** dexamethasone (formula III) is used as the oxosteroid compound.

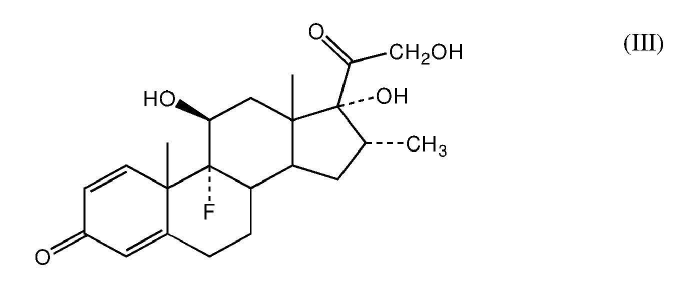

8. The process according to any of claims 1 to 6, **characterized in that** 4-androstene-3,17-dione (formula IV) is used as the oxosteroid compound.

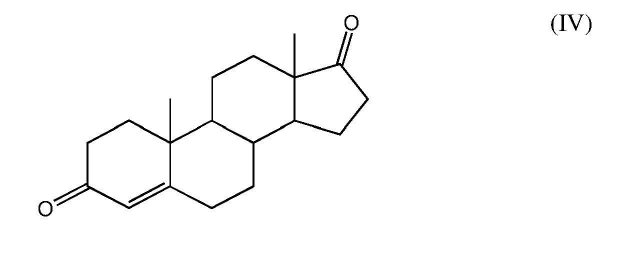

9. The process according to any of claims 1 to 6, **characterized in that** 1,4-androstadiene-3,17-dione (formula V) is used as the oxosteroid compound.

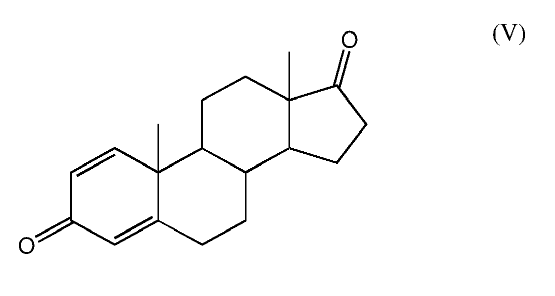

10. The process according to any of claims 1 to 6, **characterized in that** estrone (formula VI) is used as the oxosteroid compound.

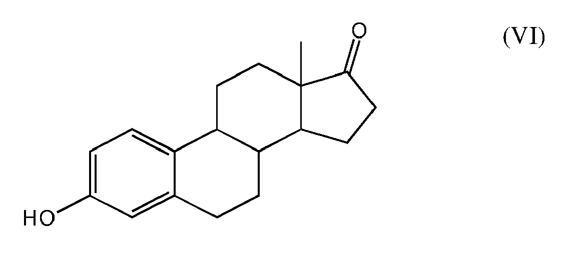

11. The process according to any of claims 1 to 6, **characterized in that** pregnenolone (formula VII) is used as the oxosteroid compound.

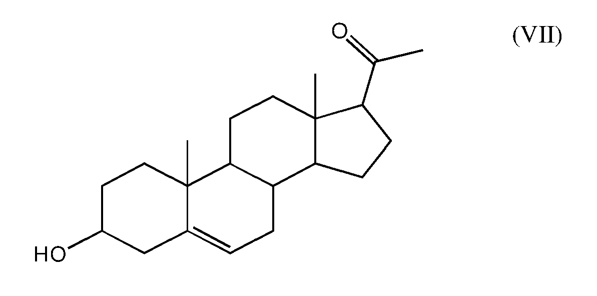

12. The process according to any of claims 1 to 6, **characterized in that** cortisone (formula VIII) is used as the oxosteroid compound.

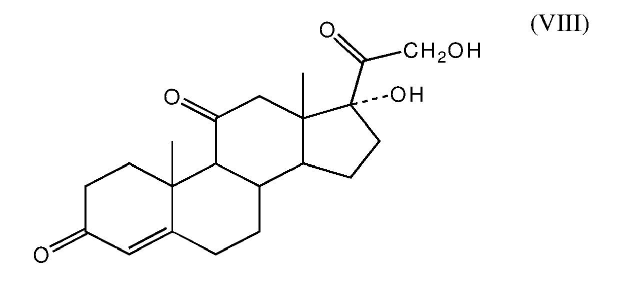

13. A process for the enzymatic oxidation of hydroxysteroid compounds of general formula I 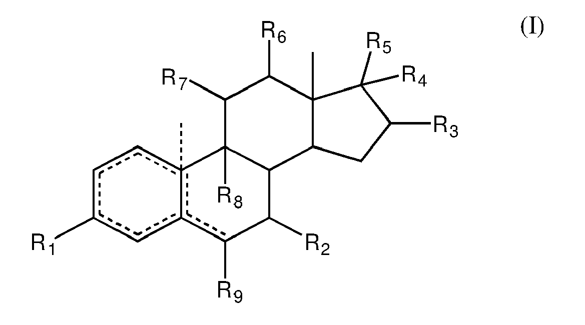 wherein
R₁ represents hydrogen, a methyl group, a hydroxy group or an oxo group,
R₂ represents hydrogen, a methyl group, an oxo group or a hydroxy group,
R₃ represents hydrogen, a hydroxy group, an oxo group or a methyl group,
R₄ represents hydrogen or a hydroxy group,
R₅ represents hydrogen, a moiety -COR₁₀, wherein R₁₀ is a C1-C4-alkyl group that is unsubstituted or substituted with a hydroxy group or a substituted or unsubstituted C1-C4-carboxyalkyl group,
or R₄ and R₅ together represent an oxo group,
R₆ represents hydrogen, a methyl group, an oxo group or a hydroxy group,
R₇ represents hydrogen, a methyl group, an oxo group or a hydroxy group,
R₈ represents hydrogen, a methyl group or a halide, and
R₉ represents hydrogen, a methyl group, a hydroxy group, an oxo group or a halide, wherein at least one of R₁, R₂, R₄, R₆, R₇, R₈ or R₉ is a hydroxy group, and the structural element 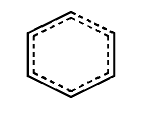
represents a benzene ring or a C6-ring having 0, 1 or 2 C-C-double bonds;
or of the bile acid derivatives cholic acid, chenodeoxycholic acid, 12-oxocholic acid, 3-hydroxy-12-oxocholic acid, ketolithocholic acid and lithocholic acid,
wherein the hydroxysteroid compound is oxidized with a hydroxysteroid dehydrogenase in the presence of a cofactor NAD or NADP, **characterized in that**
a) the hydroxysteroid compound is provided in the reaction at a concentration of ≥ 50 g/l,
b) the reduced cofactor NADH or NADPH formed by the hydroxysteroid dehydrogenase is regenerated continuously by reduction of a keto compound of general formula R_{X}R_{Y}CO, wherein Rₓ, R_{Y} independently represent a branched or unbranched C1-C8-alkyl and Cₜₒₜₐₗ≥ 3, or by reduction of a C4-C6-cycloalkanone, and
c) an additional oxidoreductase/alcohol dehydrogenase is used for the reduction of the keto compound of general formula R_{X}R_{Y}CO or of the cycloalkanone, respectively.

14. The process according to claim 13, **characterized in that** acetone, 2-butanone, 2-pentanone, 4-methyl-2-pentanone, 2-hexanone, 2-heptanone, 5-methyl-2-hexanone or 2-octanone is used as the ketone of general formula R_{X}R_{Y}CO, and cyclohexanone is used as the cycloalkanone.

15. The process according to claim 13 or 14, **characterized in that** it is carried out in an aqueous organic two-phase system.

16. The process according to claim 15, **characterized in that** an organic solvent such as, for example, diethyl ether, tertiary butyl methyl ether, diisopropyl ether, dibutyl ether, ethyl acetate, butyl acetate, heptane, hexane or cyclohexane is additionally employed.

17. The process according to any of claims 13 to 16, **characterized in that** the TTN, i.e., total turn over number = mol of oxidized hydroxysteroid compound / mol of cofactor used, is ≥10³.

18. The process according to any of claims 13 to 17, **characterized in that** at least 50% of the employed hydroxysteroid compound is oxidized to the oxosteroid compound within 2 to 96 h.

## Revendications

1. Procédé de réduction enzymatique énantiosélective de composés d'oxostéroïde de formule générale 1 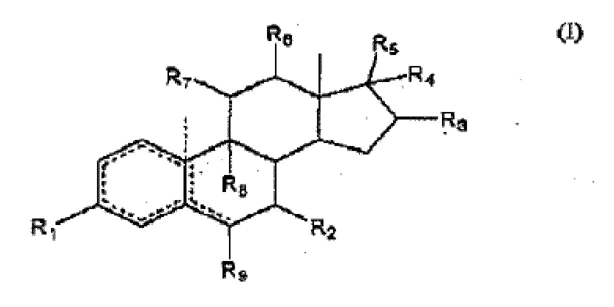 dans laquelle
R₁ représente l'hydrogène, un groupe méthyle, un groupe hydroxyle ou un groupe oxo,
R₂ représente l'hydrogène, un groupe méthyle, un groupe hydroxyle ou un groupe oxo,
R₃ représente l'hydrogène, un groupe hydroxyle, un groupe oxo ou un groupe méthyle,
R₄ représente l'hydrogène ou un groupe hydroxyle,
R₅ représente l'hydrogène, un groupe -COR₁₀ dans lequel R₁₀ est un groupe alkyle en C₁ à C₄ non substitué ou substitué avec un groupe hydroxyle ou un groupe carboxyalkyle en C₁ à C₄ substitué ou non substitué,
ou R₄ et R₅ forment ensemble un groupe oxo,
R₆ représente l'hydrogène, un groupe méthyle, un groupe hydroxyle ou un groupe oxo,
R₇ représente l'hydrogène, un groupe méthyle, un groupe hydroxyle ou un groupe oxo,
R₈ représente l'hydrogène, un groupe méthyle ou un halogénure et
R₉ représente l'hydrogène, un groupe méthyle, un groupe hydroxyle ou un halogénure,
au moins l'un des groupes R₁, R₂, R₄ + R₅, R₆, R₈ ou R₉ représente un groupe oxo et R₅ représente un groupe -COR₁₀ et l'élément structurel 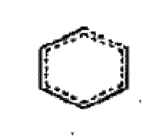
représente un cycle de benzène ou un cycle en C₆ comptant 0,1 ou 2 doubles liaisons C-C,
ou de formule II (acide cétolithocholique) 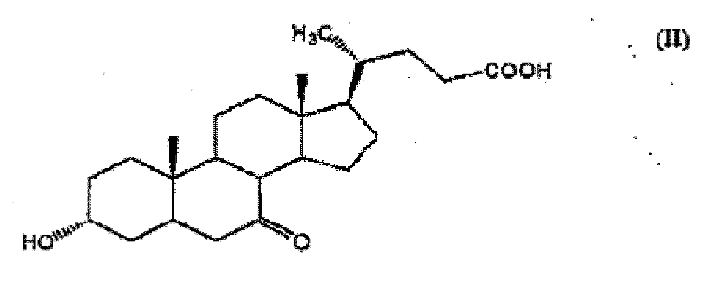
le composé d'oxostéroïde étant réduit à l'aide d'une hydroxystéroïde déshydrogénase en présence d'un cofacteur NADH ou NADPH,
**caractérisé en ce que**
a) le composé d'oxostéroïde est présent dans le milieu de réaction à une concentration ≥ 50 g/l,
b) le cofacteur oxydé NAD ou NADP formé par l'hydroxystéroïde déshydrogénase est régénéré en continu par oxydation d'un alcool secondaire de formule générale RₓR_{y}CHOH dans laquelle Rₓ et R_{y} représentent indépendamment l'un de l'autre un groupe alkyle en C₁ à C₈, ramifié ou non ramifié et C_{global} ≥ 3 ou par oxydation d'un cycloalcanol en C₄ à C₆ et
c) pour l'oxydation de l'alcool secondaire de formule générale R_{X}R_{Y}CHOH ou du cycloalcanol, on utilise une oxydoréductase-alcool déshydrogénase supplémentaire.

2. Procédé selon la revendication 1, **caractérisé en ce que** comme alcool secondaire de formule générale RₓR_{y}CHOH, on utilise le 2-propanol, le 2-butanol, le 2-pentanol, le 4-méthyl-2-pentanol, le 2-hexanol, le 2-heptanol, le 5-méthyl-2-hexanol ou le 2-octanol et comme cycloalcool un cyclohexanol.

3. Procédé selon les revendications 1 ou 2, **caractérisé en ce qu'**il est conduit dans un système aqueux organique biphasique.

4. Procédé selon la revendication 3, **caractérisé en ce qu'**il utilise en supplément un solvant organique, par exemple l'éther de diéthyle, l'éther de méthyle et de butyle tertiaire, l'éther de diisopropyle, l'éther de dibutyle, l'acétate d'éthyle, l'acétate de butyle, l'heptane, l'hexane ou le cyclohexane.

5. Procédé selon l'une des revendications 1 à 4, **caractérisé en ce que** le TTN, c'est-à-dire le "total turnover number" - nombre total de remplacements = mole de composé d'oxostéroïde réduit/mole de cofacteur consommé, est ≥ 10³.

6. Procédé selon l'une des revendications 1 à 5, **caractérisé en ce qu'**en l'espace de 2 à 96 h, au moins 50 % du composé oxostéroïde utilisé sont réduits en composé d'hydroxystéroïde.

7. Procédé selon l'une des revendications 1 à 6, **caractérisé en ce qu'**il utilise comme composé d'oxostéroïde le dexaméthasone (formule III).

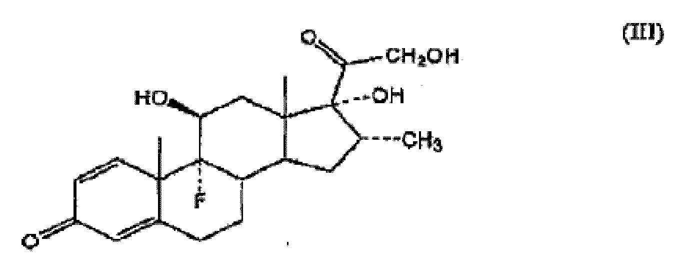

8. Procédé selon l'une des revendications 1 à 6, **caractérisé en ce que** comme composé d'oxostéroïde, il utilise la 4-androstèn-3,17-dione (formule IV).

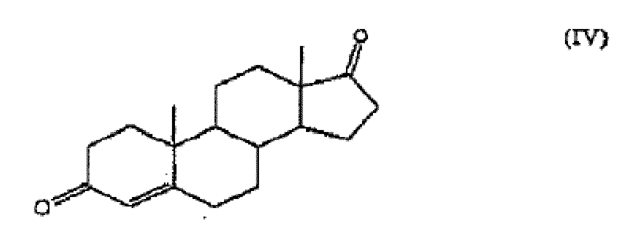

9. Procédé selon l'une des revendications 1 à 6, **caractérisé en ce que** comme composé d'oxostéroïde, il utilise la 1,4-androstadiène-3,17-dione (formule V).

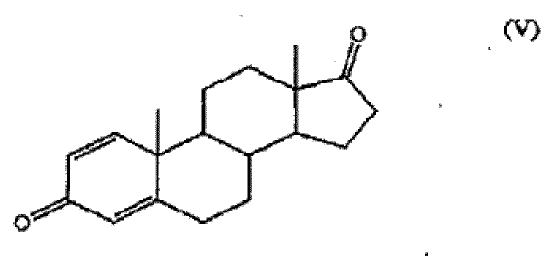

10. Procédé selon l'une des revendications 1 à 6, **caractérisé en ce que** comme composé d'oxostéroïde, il utilise l'estrone (formule VI).

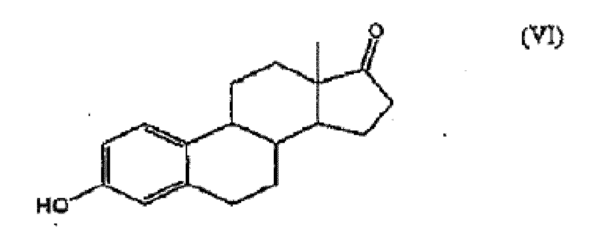

11. Procédé selon l'une des revendications 1 à 6, **caractérisé en ce que** comme composé d'oxostéroïde, il utilise le pregnenolon (formule VII).

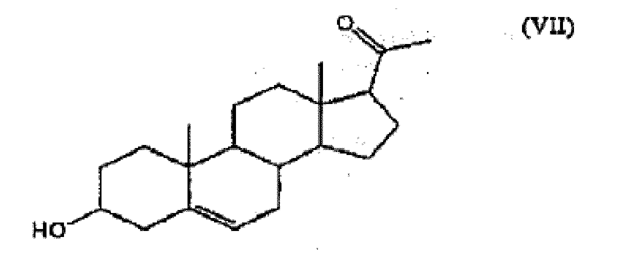

12. Procédé selon l'une des revendications 1 à 6, **caractérisé en ce que** comme composé d'oxostéroïde, il utilise la cortisone (formule VIII).

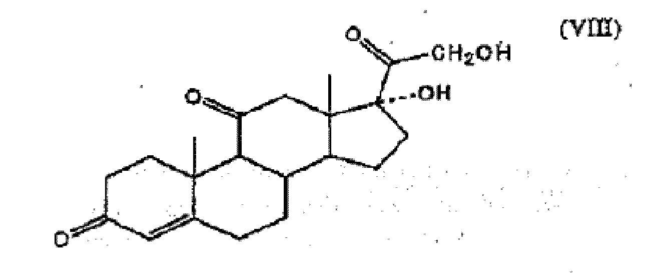

13. Procédé d'oxydation enzymatique de composés d'hydroxystéroïde de formule générale I 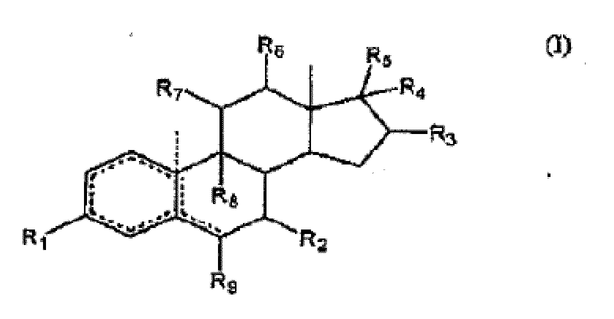 dans laquelle
R₁ représente l'hydrogène, un groupe méthyle, un groupe hydroxyle ou un groupe oxo,
R₂ représente l'hydrogène, un groupe méthyle, un groupe oxo ou un groupe hydroxyle,
R₃ représente l'hydrogène, un groupe hydroxyle, un groupe oxo ou un groupe méthyle,
R₄ représente l'hydrogène ou un groupe hydroxyle,
R₅ représente l'hydrogène, un groupe -COR₁₀ dans lequel R₁₀ est un groupe alkyle en C₁ à C₄ non substitué ou substitué avec un groupe hydroxyle ou un groupe carboxyalkyle en C₁ à C₄ substitué ou non substitué,
ou R₄ et R₅ forment ensemble un groupe oxo,
R₆ représente l'hydrogène, un groupe méthyle, un groupe oxo ou un groupe hydroxyle,
R₇ représente l'hydrogène, un groupe méthyle, un groupe oxo ou un groupe hydroxyle,
R₈ représente l'hydrogène, un groupe méthyle ou un halogénure et
R₉ représente l'hydrogène, un groupe méthyle, un groupe hydroxyle, un groupe oxo ou un halogénure,
au moins l'un des groupes R₁, R₂, R₄, R₆, R₇, R₈ ou R₉ représentent un groupe hydroxyle et
l'élément structurel 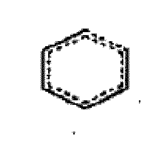
représente un cycle de benzène ou un cycle en C₆ comptant 0, 1 ou 2 doubles liaisons C-C,
ou de dérivés d'acide galénique, par exemple l'acide cholique, l'acide chénodésoxycholique, l'acide 12-oxocholique, l'acide 3-hydroxy-12-oxocholique, l'acide cétolithocholique ou l'acide litocholique,
le composé d'hydroxystéroïde étant oxydé à l'aide d'une hydroxystéroïde déshydrogénase en présence d'un cofacteur NAD ou NADP,
**caractérisé en ce que**
a) le composé d'hydroxystéroïde est présent dans le milieu de réaction à une concentration ≥ 50 g/l,
b) le cofacteur NADH ou NADPH réduit formé par la hydroxystéroïde déshydrogénase est régénéré en continu par réduction d'un composé céto de formule générale RₓR_{y}CO dans laquelle Rₓ, R_{y} représente indépendamment l'un de l'autre un alkyle en C₁ à C8 ramifié ou non ramifié et Global ≥ 3 ou par réduction d'une cycloalcanone en C₄ à C₆ et
c) pour la réduction du composé céto de formule générale R_{X}R_{Y}CO ou de la cycloalcanone, on utilise une oxydoréductase-alcool déshydrogénase supplémentaire.

14. Procédé selon la revendication 13, **caractérisé en ce que** comme cétone de formule générale R_{X}R_{Y}CO, on utilise l'acétone, la 2-butanone, la 2-pentanone, la 4-méthyl-2-pentanone, la 2-hexanone, la 2-heptanone, la 5-méthyl-2-hexanone ou la 2-octanone et comme cycloalcanone la cyclohexanone.

15. Procédé selon les revendications 13 ou 14, **caractérisé en ce qu'**il est conduit dans un système aqueux organique biphasique.

16. Procédé selon la revendication 15, **caractérisé en ce qu'**il utilise en supplément un solvant organique, par exemple l'éther de diéthyle, l'éther de méthyle et de butyle tertiaire, l'éther de diisopropyle, l'éther de dibutyle, l'acétate d'éthyle, l'acétate de butyle, l'heptane, l'hexane ou le cyclohexane.

17. Procédé selon l'une des revendications 13 à 16, **caractérisé en ce que** le TTN, c'est-à-dire le "total turnover number" - nombre total de remplacements = mole de composé d'hydroxystéroïde oxydé/mole de cofacteur utilisé, est ≥ 10³.

18. Procédé selon l'une des revendications 13 à 17, **caractérisé en ce qu'**en l'espace de 2 à 96 h, au moins 50 % du composé d'hydroxystéroïde utilisé sont oxydés en composé d'oxostéroïde.
